(19) Europäisches Patentamt
European Patent Office
Office européen des brevets

(11) Numéro de publication: **0 343 042**
**A1**

(12) # DEMANDE DE BREVET EUROPEEN

(21) Numéro de dépôt: **89401307.7**

(22) Date de dépôt: **11.05.89**

(51) Int. Cl.[4]: **C 07 C 69/38**
C 07 C 67/36

(30) Priorité: **19.05.88 FR 8806732**

(43) Date de publication de la demande:
**23.11.89 Bulletin 89/47**

(84) Etats contractants désignés:
**AT BE CH DE ES FR GB GR IT LI LU NL SE**

(71) Demandeur: **RHONE-POULENC CHIMIE DE BASE**
**25, quai Paul Doumer**
**F-92408 Courbevoie (FR)**

(72) Inventeur: **Coste, Philippe**
**112bis Cour Tolstoi**
**F-69100 Vileurbanne (FR)**

**Leconte, Philippe**
**45, rue Duquesne**
**F-69006 Lyon (FR)**

**Perron, Robert**
**La Pécolière**
**F-69390 Charly (FR)**

**Baudoin, Michel**
**Val Fontaine 35 Rue des Gouttes**
**Saint Geny les Olières F-69290 Craponne (FR)**

(74) Mandataire: **Le Pennec, Magali et al**
**Rhone-Poulenc Chimie Service Brevets Chimie 25 Quai Paul Doumer**
**F-92408 Courbevoie (FR)**

(54) **Procédé de préparation de malonates d'alkyle.**

(57) La présente invention concerne un procédé de préparation de dialkylmalonates par carbonylation de chloracétate en présence d'un catalyseur à base de chrome carbonyle et d'un alcool, cette réaction étant réalisée en milieu liquide biphasique.

Le procédé de recyclage du catalyseur fait aussi partie de l'invention.

EP 0 343 042 A1

**Description**

## PROCEDE DE PREPARATION DE MALONATES D'ALKYLE

La présente invention concerne un procédé de préparation de malonates d'alkyle. Elle concerne plus particulièrement un procédé de préparation de malonate de dialkyle.

Les malonates de dialkyle sont des composés utilisés comme intermédiaire de synthèse dans la préparation de nombreux composés phytosanitaires ou pharmaceutiques.

Ils sont préparés depuis très longtemps par exemple selon l'article décrit dans Ullmann, 1960, vol. 12, p. 192, par condensation du sel de sodium de l'acide monochloracétique avec le cyanure de sodium dans l'eau suivi d'une estérification par l'éthanol.

Ce procédé déjà ancien ne permet de fabriquer des malonates qu'à un prix de revient élevé et dans des conditions de sécurité difficiles. Aussi depuis quelques années toutes les sociétés fabriquant de malonates ont cherché à améliorer les procédés de préparation des dits malonates notamment à partir de matières premières moins coûteuses telles que l'oxyde de carbone.

Le premier procédé de synthèse de malonates à partir de chloroacétate d'éthyle et d'oxyde de carbone a été décrit dans le brevet US n° 3 116 306 dans lequel on réalise une carbonylation d'halogénure d'alkyle en présence d'un sel de cobalt tétracarbonyle de formule $Co(CO)_4M$ dans laquelle

. M représente un métal alcalin, alcalinoterreux, le zinc, le mercure, l'étain, le fer en présence d'une base organique telle que les amines tertiaires, les alcoolates de sodium et de potassium. Il est précisé dans ce texte que les hydroxydes alcalins peuvent être utilisés mais qu'ils réagissent avec l'halogénure d'alkyle en formant des produits secondaires indésirables.

Certains procédés basés sur le procédé précédemment mentionné ont été décrits tel que notamment dans le brevet FR 2 253 006 où le catalyseur de carbonylation est le cobalt carbonyle à la place du sel décrit dans le brevet US 3 116 306.

Il est aussi décrit dans le brevet FR 2 313 345 un procédé de préparation de malonates à partir de chloroacétate en présence d'oxyde de carbone et d'un catalyseur contenant du cobalt, la réaction étant réalisée en milieu homogène et en présence d'une solution d'hydroxyde de sodium dans un alcool. L'utilisation de solution alcoolique d'hydroxyde alcalin produit au cours de la réaction une mole d'eau par mole de chloroacétate consommé, or dans ce genre de réaction l'eau est à éviter absolument car elle provoque l'hydrolyse des groupes esters avec formation d'acide monoalkylmalonique ou d'ester acétique qui font fortement diminuer le rendement de la réaction. La séparation du chlorure de sodium formé et le recyclage du catalyseur ne sont faciles.

Il est aussi décrit dans de nombreux brevets (US 4399300 et GB 1448646) l'utilisation d'une base organique en milieu anhydre afin d'éviter les phénomènes de saponification de l'ester. Parmi ces bases on peut citer l'éthylate de sodium. L'inconvénient d'utiliser cette base est tout d'abord son prix de revient, par ailleurs l'utilisation de quantités importantes d'éthylate détruit le malonate et le chloroacétate. En fin de réaction il y a comme dans le brevet précédent une précipitation de chlorure de sodium qui est difficile à séparer du malonate.

La présente invention a permis de résoudre les problèmes laissés par l'art antérieur, c'est-à-dire qu'elle permet de produire des malonates de dialkyle à partir de chloracétate d'alkyle dans des conditions de sécurité satisfaisantes et à un coût inférieur aux procédés de l'art antérieur, elle permet aussi une séparation facile des sels formés et du malonate et un recyclage aisé du catalyseur.

Le procédé de la présente invention consiste à préparer les malonates de dialkyle par carbonylation du chloracétate d'alkyle en présence d'un alcool aliphatique, d'oxyde de carbone et d'un catalyseur, la réaction étant caractérisée par le fait qu'elle se déroule en milieu liquide biphasique.

Il n'était pas évident à la lecture de l'art antérieur et sachant que l'hydrolyse des esters est provoquée par la présence d'eau de pratiquer une telle réaction dans un milieu biphasique contenant de l'eau. Le fait de pratiquer cette réaction en milieu biphasique permet d'utiliser des bases beaucoup moins onéreuses telles que les hydroxydes alcalins et permet en plus une séparation facile des sels formés lors de la réaction de carbonylation.

Les malonates préparés par le procédé de la présente invention sont de préférence des malonates d'alkyle dont la chaîne alkyle contient un a quatre atomes de carbone et tout particulièrement le malonate de diéthyle.

La réaction de carbonylation des chloracétates d'alkyle peut être schématisée de la façon suivante

$$ClCH_2COOR + CO + ROH \xrightarrow[Co(CO)_4^-]{MB} CH_2(COOR)_2 + MCl + BH$$

L'alcool aliphatique est choisi de préférence parmi les alcools dont la chaîne alkyle contient un à quatre atomes de carbone, on préfère utiliser l'alcool éthylique.

Le catalyseur de la réaction de carbonylation est de préférence un catalyseur à base de cobalt on préfère utiliser un sel de cobalt tétracarbonyle.

La base (MB) est choisie parmi les bases organiques ou inorganiques telles que :
- l'éthylmalonate de sodium
- les phosphates de sodium
- l'acétate de sodium
- les hydroxydes alcalins.

On préfère utiliser les hydroxydes alcalins.

Le milieu biphasique est constitué d'une phase aqueuse servant de milieu de dissolution de la base inorganique et d'une phase organique hydrophobe. Cette phase organique est choisie parmi :

les solvants aromatiques tels que :
- le toluène
- les xylènes

les solvants aliphatiques tels que :
- les alcanes
- le cyclohexane

les éthers tels que :
- le diisopropyléther
- le méthyltertiobutyléther

les cétones telles que :
- la méthylsobutylcétone

les esters tels que :
- le chloroacétate d'alkyle
- l'acétate d'éthyle
- le malonate de dialkyle

Pour une meilleure mise en oeuvre de l'invention on préfère utiliser des rapports molaires de l'alcool au chloroacétate d'alkyle compris entre 0,1 et 10, et encore plus préférentiellement environ stoechiométrique. Un rapport extérieur à ces limites n'est pas exclu de l'invention mais n'apporte aucun avantage particulier.

Les concentrations des divers réactifs dans la phase organique sont notamment comprises entre 0,1 mole et 10 moles par litre pour le chloracétate ce qui permet de travailler dans le chloracétate pur. Le catalyseur à base de cobalt est de préférence utilisé selon une concentration dans la phase organique comprise entre $10^{-5}$ mole et 0,1 mole par litre. La quantité d'eau utilisée doit être suffisante pour obtenir un milieu biphasique, un rapport volumique calculé par rapport au solvant organique d'au moins 0,05 est conseillé.

La base est utilisée selon la stoechiométrie de la réaction de façon à neutraliser l'acide chlorhydrique formé.

Lorsque l'on utilise un hydroxyde alcalin il est préférable de l'injecter en continu à un pH contrôlé compris éventuellement entre 3 et 8 et encore plus avantageusement à un pH d'environ 5. Ce pH peut notamment être contrôlé par addition d'un tampon à base par exemple de phosphate.

Lorsque l'on utilise une base autre que les hydroxydes alcalins elle peut être introduite indifféremment au départ ou en continu.

L'oxyde de carbone peut être introduit pur ou en mélange avec des gaz inertes par exemple, azote, dioxyde de carbone, hydrogène en quantité inférieure ou égale à 20 % en volume, cet ajout n'apportant aucun avantage notable.

La pression réactionnelle est avantageusement comprise entre 1 et 300 bar et de préférence entre 5 et 20 bar, la température réactionnelle est de préférence comprise entre la température ambiante et 150°C et tout particulièrement entre 50 et 100°C.

En fin de réaction, on neutralise à pH inférieur ou égal à 7 le milieu réactionnel, on sépare les chlorures minéraux et les sels d'acides carboxyliques en phase aqueuse par simple décantation. Les sels d'acides carboxyliques peuvent être recyclés pour une nouvelle estérification.

Le sel de cobalt tétracarbonyle se trouve toujours dans la phase organique avec le malonate d'alkyle. Pour rendre le procédé économique il est nécessaire d'extraire le catalyseur de ce milieu organique afin de le recycler pour une nouvelle opération. Ce recyclage fait aussi partie de l'invention. Il est réalisé en 4 étapes.

La 1ère étape de recyclage du catalyseur consiste à l'extraire de la phase organique par oxydation sous forme de sel de cobalt (II) soluble dans un milieu aqueux. Cette oxydation du cobalt est facilement réalisée par un acide fort par exemple halogéné selon la réaction :

$MCo(CO)_4 + 3H^+ \rightarrow Co^{++} + 4CO + M^+ + d/2H_2$.

Cette réaction est réalisée de préférence en présence de chloroacétate selon la réaction

$3ClCH_2COOEt + 2Co^-(CO_4) + 3H^+ \rightarrow 2Co^{++} + 8CO + 3Cl^- + 3CH_3COOEt$

La phase organique contient après extraction du catalyseur, les matières organiques : chloracétate, malonate qui sont séparées par toute technique connue de l'homme de l'art dont la distillation.

La 2ème étape du procédé de recyclage du cobalt consiste à extraire le cobalt à son état d'oxydation (+ 2) contenu dans la phase aqueuse par un acide lourd (AH) ayant 6 à 20 atomes de carbone. Le cobalt est transformé en dicarboxylate de cobalt ($CoA_2$) en présence d'une base inorganique alcaline selon la réaction suivante :

$Co^{++} + 2\ AH + 2\ M(OH) \rightarrow CoA_2 + 2M^+ + 2H_2O$

dans laquelle :

. AH représente l'acide lourd,

. M(OH) représente la base inorganique

dans laquelle :
. M est un cation alcalin.
Le dicarboxylate de cobalt est extrait de la phase aqueuse par un solvant organique choisi parmi les dérivés aromatiques tels que :
- les xylènes,
- le toluène,
les cétones telles que :
- la méthylisobutylcétone (MIBK),
- l'acétophénone,
- la diisopropylcétone,
- la méthylisopropylcétone,
- la dibutylcétone,
les éthers tels que :
- l'éther isopropylique,
- le diphényléther,
- le méthylisobutyléther,
les alcanes tels que :
- le cyclohexane,
- le méthylcyclohexane,
les alcools contenant au moins 3 atomes de carbone tels que :
- l'isopropanol,
- le butanol,
les esters tels que :
- l'acétate d'éthyle,
- le benzoate d'alkyle,

On utilise de préférence un solvant correspondant à celui utilisé lors de la carbonylation.

L'acide lourd permettant l'extraction du cobalt est choisi comme précisé précédemment parmi les acides carboxyliques contenant 6 à 20 atomes de carbone.

On peut citer parmi ces acides :
- les acides oléiques,
- les acides palmitiques,
- les acides stéariques,
- l'acide éthyl-2 hexanoïque.

On préfère tout particulièrement utiliser l'acide éthyl-2 hexanoïque.

La 3ème étape du procédé de recyclage du cobalt consiste à réduire le cobalt de son état d'oxydation (+ 2) à l'état d'oxydation (0) par un mélange d'hydrogène et d'oxyde de carbone selon la réaction :

$2CoA_2 + 8CO + 2H_2 \rightarrow Co_2(CO)_5 + 4AH$.

Cette réaction est réalisée à l'aide d'un mélange oxyde de carbone-hydrogène contenant de préférence un rapport molaire $CO/H_2$ compris entre 0,1 et 10 et en présence de Co° initiateur, $Co_2(CO)_8$, provenant par exemple d'une opération précédente en quantité comprise entre 0,1 et 10 % molaire par rapport au $Co^{++}$.

La réduction est réalisée notamment en ce qui concerne les conditions opératoires à une température comprise entre la température ambiante et 150° C. La pression réactionnelle est de préférence comprise entre 1 et 300 bar.

Le cobalt octacarbonyle où le cobalt est à l'état d'oxydation (0) en solution dans un solvant organique est dismuté dans une 4ème étape en cobaltate (-I) tétracarbonyle et sel de cobalt à l'état d'oxydation (+ 2) par addition d'une base inorganique alcaline de préférence l'hydroxyde de sodium selon la réaction :

$2AH + 3/2Co_2(CO)_8 + 2M(OH) \rightarrow 2MCo(CO)_4 + CoA_2 + 2H_2O + 4CO$

Le sel de cobaltate (-I) tétracarbonyle, catalyseur de carbonylation est facilement séparable en phase aqueuse du milieu par décantation, il est alors introduit en présence des composés à carbonyler pour une nouvelle carbonylation réalisée en milieu biphasique.

La présente invention sera plus complètement décrite à l'aide de l'exemple suivant qui ne doit pas être considéré comme limitatif de l'invention.

EXEMPLE

Dans un réacteur de 100 l en hastelloy $B_2^R$, on charge successivement
- 5,6 kg d'une solution à 28 % d' $H_3PO_4$
- 14,16 kg d'éthanol
- 19,9 kg de toluène.

Le PH est ramené à 7 à l'aide de soude. On inerte par 3 fois 5 bar d'azote puis 3 fois 5 bar de CO, on introduit ensuite 6 kg d'une solution de $NaCo(CO)_4$ [3,5 mol de $Co^{-1}$].

Le réacteur est chauffé à 70° sous 10 bar de CO, puis on introduit 30 kg de chloracétate d'éthyle à débit constant en 2 h 15. Pendant la réaction, le PH du mélange réactionnel est maintenu à 5,3 par addition du NaOH 30 %. Dès la fin de l'introduction du chloracétate d'éthyle, le mélange réactionnel est refroidi à 40° C sous 10 bar de CO, puis ramené à PH 7 par addition de NaOH 30 %. Après détente de CO gazeux à pression atmosphérique, le mélange est décanté en deux phases.

La phase organique est traitée dans un réacteur de 160 l par une solution de HCl 38 % jusqu'à ce qu'on ne détecte plus la libération de CO par analyse en ligne (PH = 3,8-4).

On obtient une phase aqueuse contenant $CoCl_2$ et une phase organique incolore contenant 31,5 kg de diéthylmalonate. Soit un rendement malonate/chloracétate = 80,5 %.

1908 g de solution de $CoCl_2$ (3,5 % en Co) provenant de l'étape d'oxydation du $Co^{-1}$ précédente sont mélangés à une solution d'acide éthyl2 hexanoïque - toluène (554 g - 302 g).

On coule sur ce mélange une solution de NaOH 30 % jusqu'à pH 6. A la fin de la coulée, on décante 944,9 g de phase organique violette contenant 6,7 % de cobalt. La phase aqueuse résiduaire (2136 g) contient 2000 ppm de cobalt.

La phase organique d'éthyl2 hexanoate de cobalt précédemment obtenue est engagée dans un réacteur en inox. Le mélange est purgé trois fois sous 5 bar d'azote, puis de CO. On ajoute alors 0,01 mol de $Co_2(CO)_3$ au mélange précédent, puis, on laisse réagir 2 heures à 90° sous pression $CO/H_2$ = 20 bar ($CO/H_2$ 4/1).

Après refroidissement à 50° C, on dose à l'iode le $Co_2(CO)_8$ formé.

Le rendement $Co^{\circ}/Co^{++}$ est de 80 %.

La solution de $Co_2(CO)_8$ est traitée à 80° C sous 10 bar de CO par 580 g d'une solution normale de NaOH. On laisse réagir en dégazant en continu jusqu'à ce que la pression du réacteur soit constante à P = 3 bar.

Après refroidissement, on isole 700 g de solution aqueuse de $NaCo(CO)_4$ dans l'eau de titre 0,90 mol/kg. Cette solution peut être utilisée à la carbonylation du chloracétate d'éthyle en diethylmalonate.

La phase organique finale est recyclée à l'extraction du $CoCl_2$.

**Revendications**

1 - Procédé de préparation de malonate de dialkyle par carbonylation du chloracétate d'alkyl en présence d'alcool aliphatique, d'oxyde de carbone et d'un catalyseur sous forme d'un complexe cobalt-carbonyle caractérisé en ce que la carbonylation est effectuée en milieu liquide biphasique.

2 - Procédé selon la revendication 1 caractérisé en ce que la carbonylation est effectuée dans un milieu constitué d'eau et d'un solvant choisi parmi les solvants aprotiques tels que le toluène, la méthylisobutylcétone, l'acétate d'éthyle, les xylènes, le cyclohexane et ses dérivés, les éthers, le chloracétate d'alkyle, le malonate d'alkyle.

3 - Procédé selon la revendication 2 caractérisé en ce que la carbonylation est effectuée dans un solvant constitué d'eau et de toluène.

4 - Procédé selon la revendication 1 caractérisé en ce que la carbonylation est effectuée dans un milieu contenant au moins une base choisie parmi l'éthylmalonate de sodium, les phosphates sodiques, l'acétate de sodium, les hydroxydes alcalins.

5 - Procédé selon la revendication 1 caractérisé en ce que l'alcool aliphatique contient 1 à 4 atomes de carbone.

6 - Procédé selon la revendication 1 et la revendication 4 caractérisé en ce que le PH est maintenu entre 3 et 8 au cours de la réaction par addition continue de soude aqueuse.

7 - Procédé selon la revendication 1 caractérisé en ce que la carbonylation est effectuée à une température comprise entre la température ambiante et 150° C et de préférence entre 50 et 100° C.

8 - Procédé selon la revendication 1 caractérisé en ce que la carbonylation est effectuée sous une pression comprise entre 1 et 300 bar et de préférence entre 5 et 20 bar.

9 - Procédé de recyclage du complexe cobalt carbonyle utilisé lors d'une carbonylation réalisée en milieu biphasique solvant organique-eau caractérisé en ce que

dans une première étape on oxyde le cobalt par addition d'un acide fort

dans une deuxième étape on extrait le cobalt oxydé (Co II) par un acide carboxylique lourd contenant 6 à 20 atomes de carbone dans un solvent organique et en présence d'un hydroxyde alcalin

dans une troisième étape on réduit le carboxylate de cobalt par un mélange d'hydrogène et d'oxyde de carbone en présence de Co° comme initiateur

dans une quatrième étape on dismute le cobalt réduit par addition d'une base inorganique et on extrait en phase aqueuse le cobaltate tétracarbonyle.

10 - Procédé selon la revendication 9 caractérisé en ce que dans la première étape l'oxydation est réalisée par l'acide chlorhydrique.

11 - Procédé selon la revendication 9 caractérisé en ce que dans la deuxième étape l'acide lourd est l'acide éthyl-2 hexanoïque.

12 - Procédé selon la revendication 9 caractérisé en ce que dans la deuxième étape le solvant organique est choisi parmi les dérivés aromatiques, les esters, les cétones, les éthers aliphatiques ou aromatiques, les alcanes.

13 - Procédé selon la revendication 12 caractérisé en ce que le solvant est le toluène.

14 - Procédé selon la revendication 9 caractérisé en ce que dans la troisième étape la réduction est effectuée par un mélange oxyde de carbone-hydrogène contenant un rapport molaire hydrogène/oxyde de carbone compris entre 0,1 et 10.

15 - Procédé selon la revendication 14 caractérisé en ce que la réduction est effectuée à une température comprise entre la température ambiante et 150° C.

16 - Procédé selon la revendication 14 caractérisé en ce que la réduction est effectuée à une pression comprise entre 1 et 300 bar.

17 - Procédé selon la revendication 14 caractérisé en ce que la dismutation est effectuée par addition d'une solution aqueuse de soude.